# EUROPEAN PATENT SPECIFICATION

(11) **EP 2 455 031 B1**
(45) Date of publication and mention of the grant of the patent: **15.01.2014**
(21) Application number: 11190280.5
(22) Date of filing: 23.11.2011
(51) Int. Cl.: A61B 17/70

(54) **Head to head connector for bone fixation assemblies**
Kopfsteckverbinder für Knochenfixierungsanordnungen
Connecteur tête-à-tête pour les ensembles de fixation osseuse

(30) Priority: 23.11.2010 US 416460 P
(43) Date of publication of application: 23.05.2012
(73) Proprietor: Aesculap Implant Systems, LLC, Center Valley, PA 18034 (US)
(72) Inventor: Haskins, Tyler, Bethlehem, PA Pennsylvania 18018 (US); Wing, Charles, Center Valley, PA Pennsylvania 18034 (US)
(74) Representative: Winter, Brandl, Fürniss, Hübner, Röss, Kaiser, Polte - Partnerschaft

(56) References cited:
- EP-A1- 0 514 303
- US-A1- 2002 007 183
- US-A1- 2007 270 809
- US-A1- 2010 087 867
- US-A1- 2011 046 675
- US-B2- 7 645 294

## Description

### FIELD

The present invention relates generally to bone fixation assemblies and more specifically to a reinforcing cross-connector apparatus.

### BACKGROUND

When performing posterior cervical stabilization, a surgeon may place screws into the lateral mass of the cervical vertebral body, followed by a fixation element, such as a titanium rod. The titanium rod may be received in openings that pass through the heads of the screws, and may be secured in the openings with set screws. A pair of rods may be secured to the spine in a longitudinal arrangement, each rod extending generally parallel to one another. This construct stabilizes the cervical spine to aid in fusion of one or more levels. Occasionally, the condition of the spine requires a more rigid construct to stabilize the spine. In these situations, a transverse connecting element may be used to interconnect the two rods, like a bridge, to add stability to the construct. The transverse connecting element may be attached directly to the rod extending on one side of the spine and spanned to connect directly to the rod on the opposing side. This connecting element is sometimes referred to as a "rod-to-rod connector", insofar as it interconnects two rods to one another.

US 7,628,799 B2 and US 7,744,632 B2 show a number of rod-to-rod connectors that clamp directly onto spinal fixation rods. Although these rod-to-rod connectors increase the overall rigidity of construct, they pose the risk of creating stress points along the fixation rods that can affect the integrity of the rods over time.

US 7,645,294 B2 shows another type of transverse connecting element that attaches directly to the heads of bone screws, instead of the rods. This type of transverse connector is sometimes called a "head-to-head connector", insofar as it interconnects the heads of two pedicle screw assemblies. Although head-to-head connectors reduce the concern for placing stress on the rods, head-to-head connectors create other concerns. The ability to use head-to-head connectors depends in large part on the location of the bone screws. Bone screws can be positioned relative to one another with different spacings and angular orientations.

Head-to-head connectors often have fixed geometries that cannot be used unless they fit precisely over the bone screws. In addition, head-to-head connectors require separate set screws, caps, or other hardware to connect them with bone screw assemblies. This extra hardware adds to the number of small components that must be sterilized and handled. Moreover, head-to-head connectors often require at least two locking steps on each bone screw, for a total of at least four steps. In US 7,645,294 B2, for example, a set screw must be secured to the bone screw in a first step, and a cap must be secured over a plate and the set screw in a second step. Each of these steps requires the surgeon to precisely thread a very small component to another small component. These threading steps increase the difficulty of the procedure, and if done improperly, can lead to jammed threads and damage to the plates, set screws and caps.

US 2010/0087867 A1 discloses a fastener assembly including an installed pedicle screw including a fastener head to which a prosthetic member is secured, a fastening portion fastened to the fastener head over the prosthetic member, and a connector element that extends from the fastening portion, the connector element being connected to another spinal structure.

US 2011/0046675 A1 discloses a transverse rod connector including an elongate member having first and second ends and first and second connection members. The first and second connection members are connected with first and second ends, respectively. The first and second connection members are configured for multidirectional positioning with respect to the elongate member. The first and second connection members are each dimensioned to be selectively and releasably secured to a bone anchor. The elongate member is longitudinally adjustable.

There are multiple competing needs that must be addressed when designing an apparatus to reinforce spinal fixation assemblies, as demonstrated by the drawbacks observed with known rod-to-rod connectors and known head-to-head connectors. Rod-to-rod connectors raise the concern of putting excessive stress on the rods. Head-to-head connectors can reduce this concern, but raise new challenges. To connect to bone screws, the head-to-head connector must accommodate the components used in the bone screw assembly, and be able to adapt to different bone screw arrangements. As a result, known head-to-head connectors can satisfy the need to reduce stress on rods, but sacrifice other equally important needs. Therefore, there is a need for improved transverse connectors that address competing needs without sacrificing one need for another.

### SUMMARY

The drawbacks of known transverse connectors, and the competing needs they serve, are addressed by an apparatus in accordance with the invention.

According to the invention, a reinforcing cross connector apparatus connects a first bone fixation assembly to a second bone fixation assembly, the first bone fixation assembly having a first bone screw and a first rod receiver encompassing a head of the first bone screw, the second bone fixation assembly having a second bone screw and a second rod receiver encompassing a head of the second bone screw. The reinforcing cross connector apparatus comprises a first cap encompassing and engaging an outer surface of the first rod receiver, the first cap having a first locking mechanism, wherein a lower portion of the first locking mechanism engages an inner surface of the first rod receiver and an upper portion of the first locking mechanism engages an inner surface of the first cap. It further comprises a second cap encompassing and engaging an outer surface of the second rod receiver, the second cap having a second locking mechanism, wherein a lower portion of the second locking mechanism engages an inner surface of the second rod receiver and an upper portion of the second locking mechanism engages an inner surface of the second cap. Further, the reinforcing cross connector apparatus comprises a connecting element which links the first cap and the second cap together, the first locking mechanism securing the first cap and connecting element to the first bone fixation assembly, and the second locking mechanism securing the second cap and connecting element to the second bone fixation assembly.

### BRIEF DESCRIPTION OF THE DRAWINGS

The foregoing summary and the following description will be better understood in conjunction with the drawing figures, of which:
FIG. 1 is a perspective view of an occipitocervical fixation assembly with a transverse connector in accordance with one exemplary embodiment of the invention;
FIG. 2 is a perspective view of two pedicle screws and fixation rods connected to the transverse connector of FIG. 1;
FIG. 3 is a cross-section view of the assembly of FIG. 2;
FIG. 4 a perspective view of two pedicle screws and fixation rods, with another transverse connector in accordance with the invention;
FIG. 5 is a cross-section view of the assembly of FIG. 4; and
FIG. 6 is an exploded perspective view of two pedicle screws and fixation rods, with another transverse connector in accordance with the invention.

### DETAILED DESCRIPTION

Although the invention is illustrated and described herein with reference to specific embodiments, the invention is not intended to be limited to the details shown. Rather, various modifications may be made in the details within the scope and range of equivalents of the claims and without departing from the invention.

The present invention relates to a reinforcing cross connector apparatus as claimed hereafter. Preferred embodiments of the invention are set forth in the dependent claims.

When used in reference to cylindrical or generally cylindrical objects, the term "longitudinal axis" as used herein means an axis connecting the center point of each cross section taken through the object, where the axis passes through the object parallel to the length of the object and perpendicular to the diameter of the object.

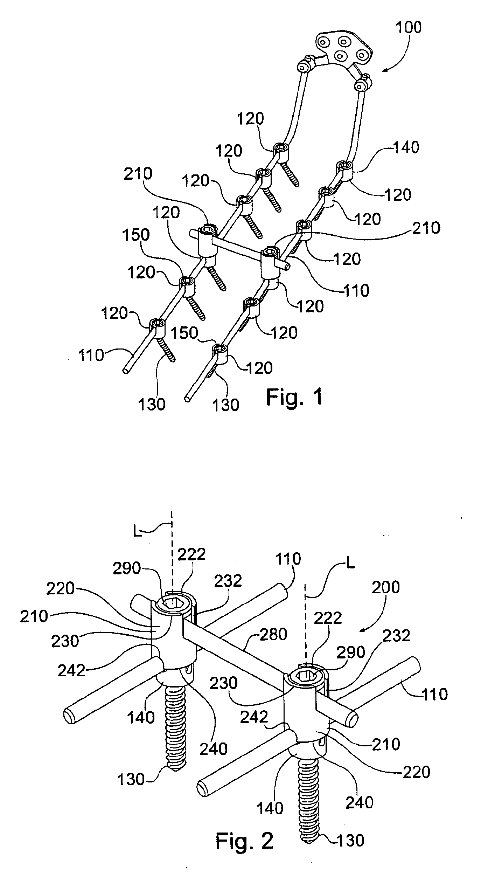

Referring to FIG. 1, an occipitocervical fixation assembly 100 is shown. Fixation assembly 100 includes a pair of elongated fixation members in the form of spinal rods 110. Each rod 110 is inserted into a series of pedicle screw assemblies 120 configured for anchoring into vertebrae. Each pedicle screw assembly 120 includes a bone screw 130, a rod receiver 140, and a securing element 150 for locking one of the rods 110 into the rod receiver.

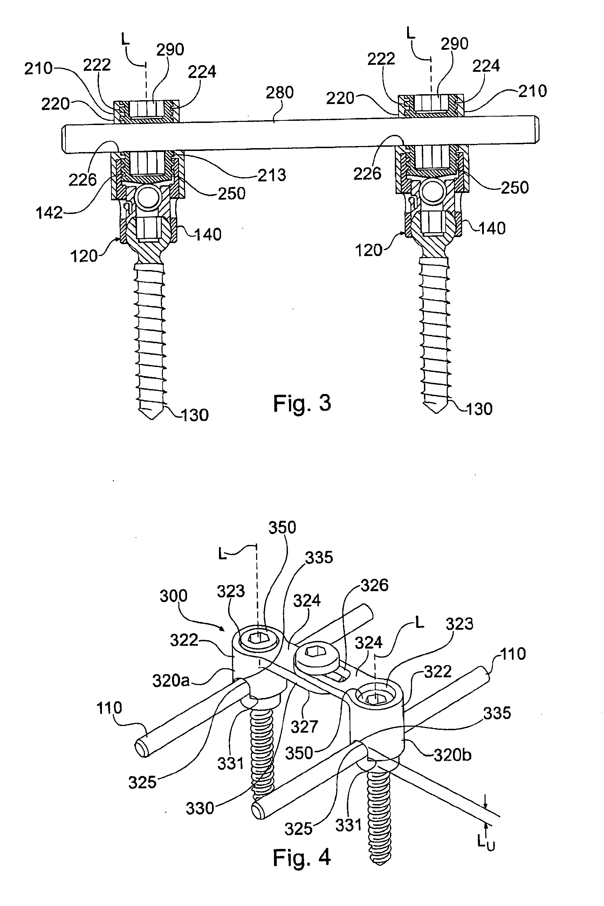

The rigidity of assembly 100 is reinforced with a head-to-head cross connector assembly 200 that bridges two pedicle screw assemblies 120. Connector assembly 200, which is shown in more detail in FIGS. 2 and 3, includes a pair of caps 210 and a transverse connecting element in the form of a rod 280. Each cap 210 includes a lower locking element 250 and an upper locking element 290. Lower locking elements 250 have a thread configuration identical to the securing elements 150 provided with the pedicle screw assemblies, and are designed to serve the same function as the securing elements 150. As such, caps 210 can be used with the securing elements 150 that are provided with the pedicle screw assemblies 120, and locking elements 250 need not be used.

Each cap 210 has a generally cylindrical body 220 that is hollow, forming a longitudinal bore 222. Bore 222 extends along the longitudinal axis L of the body 220. Each body 220 has a first end 230 adapted to receive one section of transverse connecting element 280 and one of the locking elements 290. Each first end 230 includes a pair of U-shaped channels 232 that are diametrically opposed to one another, with respect to longitudinal axis L. Each U-shaped channel 232 is positioned to receive one section of transverse connecting element 280, and orient the transverse connecting element transversely with respect to the orientation of fixation rods 110. Each body 220 has a second end 240 opposite first end 230. Each second end 240 has a pair of inverted U-shaped channels 242 that fit over the contours of rods 110.

In FIG. 3, caps 210 are shown secured to the tops of two pedicle screw assemblies 120, with the transverse connecting element 280 locked in both caps. Caps 210 are placed over the pedicle screws with their U-shaped channels 232 aligned with one another to form a continuous passage 226 to receive the transverse connecting element 280. The bore 222 in each cap 210 includes an internal thread 224. Each locking element 290 has an external thread configured to mate with an internal thread 224 in either of caps 220. In this configuration, each locking element 290 is configured to be driven into the bore of one of the caps 210 to lock a section of transverse connecting element 280 into the cap, above the locking element 250.

Connector assembly 200 may be secured to occipitocervical fixation assembly 100 in the following manner. After rods 110 are secured in the two rows of pedicle screw assemblies 120, one or more locations along the construct are selected for placement of a connector assembly 200. The number of transverse connector assemblies that are used may vary depending on the amount of reinforcement that is desired. For this example, it is assumed that only one transverse connector assembly is needed to reinforce the entire construct.

To select a location for transverse connector assembly 200, an opposing pair of pedicle screw assemblies is selected that will support the transverse connector assembly. The location may be chosen based on the number and arrangement of pedicle screws, the condition of the bones, and other factors. In FIG. 1, transverse connector assembly 200 is placed at a central location of the construct, so as to bridge rods 110 at or close to their midpoints.

Once a location for transverse connector assembly 200 is selected, the securing elements 150 inside the chosen pedicle screw assemblies 120 can be removed and replaced with locking elements 250. Alternatively, securing elements 150 can be left in place. A cap 210 is placed over the rod receiver 140 of each of the selected pedicle screw assemblies 120. The locking element 250 in each cap 210 is then threaded into the rod receiver 140 and driven downwardly by a driver to securely connect the cap to the rod receiver. The internal thread 224 in each cap matches an internal thread configuration 142 inside the corresponding rod receiver 140. When a cap 210 is placed over a rod receiver 140, the internal threads 142 and 224 align to form a continuous thread pattern with a smooth transition 213. Securing elements 250 are driven downwardly until they straddle the adjoining thread configurations 142 and 224 and overlap the transition 213. In this arrangement, securing elements 250 securely connect the caps 210 to their corresponding rod receivers 140. The internal thread configurations 142 and 224 and dimensions of the components are chosen so that each securing element 250 is completely advanced, or "bottoms out", at a position in the rod receiver where it straddles the internal thread configurations and contacts the associated rod 110. Each securing element 250 bottoms out and secures the cap 210 in a condition where the perimeter edge inside each inverted U-shaped channel 242 firmly engages the contour of the associated rod 110.

Once the caps 210 are secured over the rod receivers 140, rod 280 is placed into the U-shaped channels 232 of each cap to bridge the two rod receivers. A locking element 290 is then inserted into the bore 222 of each cap 210 and driven downwardly with a driver until the locking element contacts rod 280. Each locking element 290 is driven against the rod 280 to urge the rod downwardly into a firmly seated position in the U-shaped channels 232 of the cap 210. At this stage, transverse connector assembly 200 is secured in place and reinforces the construct.

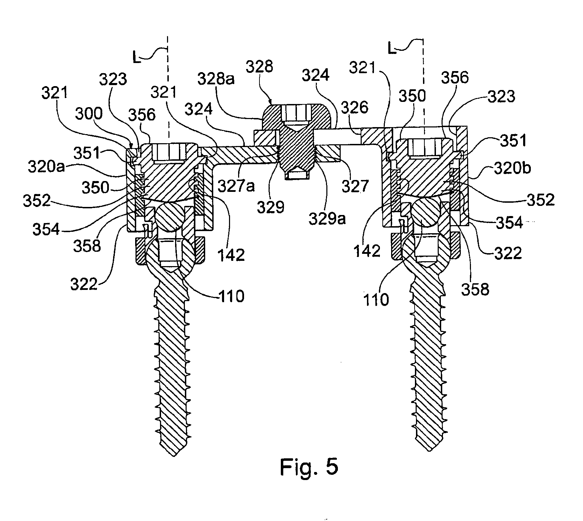

Referring to FIGS. 4 and 5, a connector assembly 300 in accordance with another exemplary embodiment of the invention is shown. Connector assembly 300 includes a pair of caps 320a and 320b, and an integrated adjustable linkage 330 that interconnects the caps. Connector assembly 300 also includes single-step locking mechanisms 350 in the form of built-in locking elements that are captively retained in each of the caps 320a and 320b.

Caps 320a and 320b each include a generally cylindrical body 322 having a hollow bore 323 that extends along the longitudinal axis L of the body. Caps 320a and 320b also include a link portion 324 that extends from the cap in a direction generally perpendicular to the longitudinal axis L of the body. The axial length of cap 320b is greater than the axial length of cap 320a, as shown in FIG. 5. In this arrangement, link portion 324 of cap 320b is positioned to overlap or rest on top of link portion 324 of cap 320a, when the two are assembled. Link portion 324 on cap 320b has an elongated slot 326. Link portion 324 on cap 320a has a hole 327 with an internal thread 327a. Elongated slot 326 is positioned to align with threaded hole 327 when link portion 324 for cap 320b is positioned over top of link portion 324 of cap 320a. When aligned, elongated slot 326 and threaded hole 327 form a through-passage adapted to receive a fastener 328. Fastener 328 includes fastener head 328a and a threaded shaft 329 with a thread 329a that mates with internal thread 327a in hole 327. In this arrangement, shaft 329 can be passed through slot 326 and partially screwed into threaded hole 327 to couple link portions 324 together in a loosened condition. In the loosened condition, link portions 324 can translate and pivot relative to one another. Shaft 329 can be screwed further into threaded hole 327 and turned completely to a tightened condition, in which the link portions 324 are pulled together and secured in a fixed relationship by frictional engagement. When connector assembly 300 is assembled, fastener 328 can be loosened to allow the link portions 324 to translate and pivot relative to one another, thereby permitting the spacing between caps 320a and 320b to be increased or decreased to match the spacing between the two pedicle screw assemblies 120 that are to support the assembly.

The single-step locking mechanisms 350 are configured to lock spinal fixation rods 110 to pedicle screw assemblies 120, and lock connector assembly 300 to the pedicle screw assemblies 120, in a single turning operation. That is, the single-step locking mechanism 350 can "lock down" rods 110 in rod receivers 140, and also lock connector assembly 300 to the rod receivers, in one singular motion characterized by a continuous and uninterrupted rotation of the locking element 352. This singular motion has advantages over other head-to-head connector assemblies because it reduces the number of locking steps. In addition, it avoids the tedious steps of threading small components together. After the connector assembly 300 is placed over a pair of pedicle screw assemblies 120, each locking mechanism 350 is driven into a rod receiver 140 to lock down the fixation rod 110 in the rod receiver and lock the connector assembly 300 to the corresponding pedicle screw assembly. This results in only two tightening steps to secure the connector assembly 300. In contrast, the head-to-head connector in Figure 1A of U.S. Patent No. 7,645,294 requires at least four tightening steps. By reducing the number of tightening steps, connector assemblies in accordance with the invention, like connector assembly 300, shorten the time required to install and reinforce a fixation rod assembly, and reduce the number of complications that may occur.

Each single-step locking mechanism 350 includes a threaded locking element 352 that is generally cylindrical. Locking element 352 has a first end 354 and a second end 356. First end 354 has an external thread 355 configured to engage an internal thread 142 on the interior of a rod receiver 140. In addition, first end 354 includes an end face 358. End face 358 may or may not bear against fixation rod 110 and hold the fixation rod in a locked position in the rod receiver 140. As explained in more detail below, end face 358 need not contact rod 110 because other parts of the caps may contact and secure the rod. End face 358 has a slightly cone-shaped profile. Nevertheless, locking mechanisms in accordance with the invention may include end faces with various geometries, including convexly curved, concavely-curved, cone-shaped or flat geometries.

The second end 356 of each locking mechanism 350 includes a flange 351 that extends radially outwardly. Flanges 351 are held captive in annular grooves 321 in the interior of caps 320a and 320b, so that locking mechanisms 350 cannot move axially in the caps, but are free to rotate in the caps. Flanges in accordance with the invention preferably have a circular cross-section, but may have other cross-sectional configurations that extend into annular grooves.

Connector assembly 300 may be secured to an occipitocervical fixation assembly in the following manner. First, fastener 328 is inserted through elongated slot 326 of cap 320b and screwed partially into threaded hole 327 of cap 320a. The threaded shaft 329 of fastener 328 is screwed partially into hole 327, but not tightened completely, so that link portions 324 are coupled in the loosened condition. In this condition, the link portions 324 can translate and rotate relative to one another to adjust the spacing between the caps, until the spacing between the caps matches the spacing between the pedicle screw assemblies on which the connecting assembly 300 is to be placed. The caps 320a and 320b are adjusted until they align more or less with rod receivers 140. Then, any securing elements in the rod receivers 140 are removed (as they will be replaced by the single-step locking mechanisms 350). Caps 320a and 320b are then placed onto the rod receivers 140. Link portions 324 are left in the loosened condition so that the caps 320a and 320b can continue to adjust to the spacing and relative positions of the rod receivers 140. Once the caps 320a and 320b are placed on the rod receivers 140, they are advanced down over the rod receivers by rotating the single-step locking mechanisms 350. Caps 320a and 320b preferably have inverted U-shaped channels 325 to fit over the contours of the rods 110. Each U-shaped channel 325 has a rounded end 335 that engages the circumference of each rod after the cap is advanced down over the rod receiver. Once the caps 320a and 320b are completely advanced over the rod receivers, fastener 328 is tightened to lock the link portions 324 together in a fixed condition. As fastener 328 is tightened, the fastener pulls link portion 324 on cap 320a upwardly and into engagement with the underside of link portion 324 on cap 320b. This holds the two link portions 324 together in a tight frictional engagement, fixing the spacing between caps 320a and 320b.

At this stage, the fixation rods 110 rest inside rod receivers 140 but may or may not be locked in place in a fixed condition. In addition, connector assembly 300 rests on the pedicle screw assemblies 120 and interconnects the fixation rods 110 indirectly, but is not locked down onto the rod receivers 140. To lock down the fixation rods 110, and lock down connector assembly to the rod receivers 140, the locking mechanism 350 in each cap 320a and 320b is rotated so that the external thread 355 on each locking mechanism engages the internal thread 142 inside each rod receiver 140. Once the external threads 355 on locking mechanisms 350 engage the internal threads 142 in the rod receivers 140, the locking mechanisms are rotated in their respective caps. Each locking mechanism 350 is held captive in its respective cap by its flange 351, and cannot move axially in the cap. As each locking mechanism 350 is driven into a rod receiver 140, the flange 351 pulls the cap downwardly over the rod receiver and forces the rod downwardly into a seated position in the rod receiver. Once seated, the fixation rod 110 is locked in the rod receiver 140, and the connector assembly 300 is locked onto the pedicle screw assemblies 120.

In the embodiments described thus far, the caps play a number of important roles. The cylindrical geometry of the caps allows the caps to be placed over cylindrical rod receivers in a coaxial relationship. That is, the longitudinal axis passing through a cap is coextensive with the longitudinal axis of the rod receiver, when the cap is placed over the rod receiver. The section of each cap that receives the rod receiver has an inner diameter that is equal to or slightly greater than the outer diameter of each rod receiver. In this arrangement, the cap is centered over the rod receiver and can slide axially over the rod receiver in a telescoping relationship when the cap is placed over the rod receiver. Caps 320a and 320b circumscribe the rod receivers 140 and prevent the rod receivers from radially expanding or splaying when locking mechanisms 350 are advanced into the rod receivers.

Caps 320a and 320b are automatically centered in coaxial alignment with rod receivers 140 upon placing the caps over the rod receivers. As such, caps 320a and 320b further serve as centering guides to position the locking mechanisms 350 in proper alignment with inner threads 142 within rod receivers 140. This automatic centering ensures that the single-step locking mechanisms 350 readily engage inner threads 142 when the cross-connector assembly is to be locked.

Referring to FIG. 4, the U-shaped channels 325 have a length LU parallel to the longitudinal axis of their respective caps. Length LU is greater than the diameter of the rods 110 extending through the U-shaped channels 325. Because channels 325 are longer than the diameters of the rods, each cap has an extension portion 331 that extends below the rods. The axial spacing between the rounded ends 335 and the single-step locking mechanisms 350 are selected so as to control when the rod 110 is contacted by the cap during advancement of the cap over the rod receiver 140. In particular, the rounded ends 335 are located relative to the single-step locking mechanisms 350 such that the single-step locking mechanisms engage the inner threads 142 in the rod receivers 140 before the rounded ends engage the rods 110. In this arrangement, each single-step locking mechanism 350 can be screwed into a rod receiver 140 to advance the cap downwardly over the rod receiver and rod 110 before the rounded ends 335 contact the rod. Thus, caps 320a and 320b can be secured over the rod receivers 140 before they begin to push the rod 110 into a seated position in the rod receiver. After the cap is advanced past a certain point, the rounded ends 335 of U-shaped channels 325 preferably contact the rod 110. Continued rotation of the single-step locking mechanism 350 past this point will drive the rod 110 downwardly in the rod receiver 140 until the rod is in a fully seated position. Consequently, single-step locking mechanisms 350 and caps 320a and 320b serve as an integral rod-persuader unit within the cross-connector assembly that moves the rod into a final seated position.

The end face 358 of single-step locking mechanism 350 need not engage the rod 110 in those situations where the rounded ends 335 of U-shaped channels 325 contact the rod. The rounded ends 335 provide two points of contact to advance the rod 110. The end face 358 can provide a third point of contact on the rod 110, if desired, but it is not necessary to have this third point of contact if the rod is contacted by the rounded ends 335. Likewise, if end face 358 contacts the rod 110, the rounded ends 335 of channels 325 need not contact the rod. More contact points on the rod 110 are preferred, however, as they distribute forces over a larger area of the rod.

In many instances, the pedicle screws to be bridged with a cross connector assembly will not be parallel to one another. Moreover, the two rods being bridged may not be parallel to one another. In such cases, the rod receivers will not be parallel to one another, and the orientations of the caps must be adjusted accordingly. Cross connector assemblies in accordance with the invention may include any number of mechanisms to adjust the relative orientation between the caps, so as to accommodate the relative orientations of pedicle screws and rods being bridged.

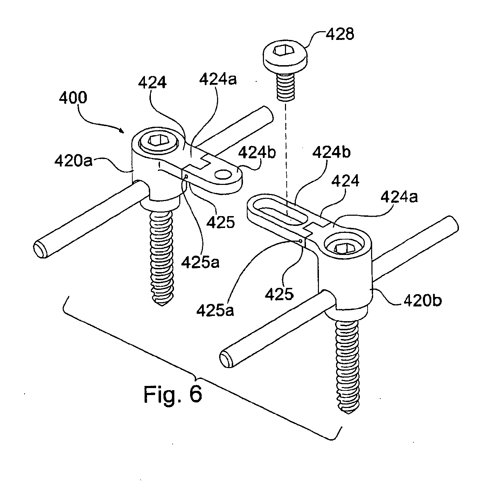

Referring now to FIG. 6, a head-to-head connector assembly 400 with an angular adjustment feature is shown in accordance with another exemplary embodiment of the invention. Assembly 400 includes a first cap 420a and a second cap 420b that are similar in many respects to the caps 320a and 320b of assembly 300. First cap 420a includes a link portion 424 with a pivot hinge 425 that divides the link portion into a fixed section 424a and a pivoting section 424b. Similarly, second cap 420b includes a link portion 424 with a pivot hinge 425 that divides the link portion into a fixed section 424a and a pivoting section 424b. Pivot hinges 425 include pin connections 425a that connect the fixed sections 424a with the pivoting sections 424b. In this arrangement, link portions 424 allow caps 420a and 420b to translate, rotate and pivot relative to one another to accommodate not only a specific distance between bone screw assemblies, but also adjust to the relative orientations of bone screw assemblies where the screw heads are not parallel to one another. Pivot hinges 425 preferably exhibit a high amount of friction and require substantial force to move pivoting sections 424b, so that the pivoting sections resist movement after they are adjusted to the desired angle. Alternatively, hinges 425 may include any known type of locking mechanism to hold the pivoting sections 424b in a fixed position after adjustment.

Although FIG. 6 shows pin connections, assemblies in accordance with the invention may include other types of hinges and couplings, such as plastically deformable link portions or other bending structures that allow the link portions to pivot or bend about one axis. Alternatively, the link portions may incorporate a universal coupling or ball joint so that each cap can move polyaxially with respect to its respective fastener, allowing each cap to be adjustable relative to multiple axes. Polyaxial motion allows the cap to adjust to many combinations of rod receiver orientations and positions. As noted above, link portions and caps may include a variety of locking mechanisms for locking the position of the pivoting sections after they are adjusted.

## Claims

1. A reinforcing cross connector apparatus (100, 200, 300) which connects a first bone fixation assembly to a second bone fixation assembly, the first bone fixation assembly having a first bone screw (130) and a first rod receiver (140) encompassing a head of the first bone screw (130), the second bone fixation assembly having a second bone screw (130) and a second rod receiver (140) encompassing a head of the second bone screw (130), the reinforcing cross connector apparatus (200, 300, 400) comprising:
a first cap (210, 320a, 420a) encompassing and engaging an outer surface of the first rod receiver (140), the first cap (210, 320a, 420a) having a first locking mechanism, wherein a lower portion (250, 354) of the first locking mechanism engages an inner surface of the first rod receiver (140) and an upper portion (290, 356) of the first locking mechanism engages an inner surface of the first cap (210, 320a, 420a);
a second cap (210, 320b, 420b) encompassing and engaging an outer surface of the second rod receiver (140), the second cap (210, 320b, 420b) having a second locking mechanism, wherein a lower portion (250, 354) of the second locking mechanism engages an inner surface of the second rod receiver (140) and an upper portion (290, 356) of the second locking mechanism engages an inner surface of the second cap (210, 320b, 420b); and
a connecting element (280, 324) which links the first cap (210, 320a, 420a) and second cap (210, 320b, 420b) together, the first locking mechanism securing the first cap (210, 320a, 420a) and connecting element (280, 324) to the first bone fixation assembly, and the second locking mechanism securing the second cap (210, 320b, 420b) and connecting element (280, 324) to the second bone fixation assembly.

2. The reinforcing cross connector apparatus of claim 1, wherein
the first cap (210, 320a, 420a) includes a bore (222, 323) defined therein, a longitudinal axis (L) extends through the bore (222, 323) of the first cap (210, 320a, 420a), and a first end (230) of the first cap (210, 320a, 420a) receives the first locking mechanism therein;
the second cap (210, 320b, 420b) includes a bore (222, 323) defined therein, a longitudinal axis (L) extends through the bore (222, 323) of the second cap (210, 320b, 420b), and a first end (230) of the second cap (210, 320b, 420b) receives the second locking mechanism therein;
the connecting element (280, 324) linking the first end (230) of the first cap (210, 320a, 420a) and the first end (230) of the second cap (210, 320b, 420b) together; and
a second end (240) of the first cap (210, 320a, 420a) which is disposed opposite the first end (230) of the first cap (210, 320a, 420a) defines a socket, which receives therein the first rod receiver (140) of the first bone fixation assembly, and a second end (240) of the second cap (210, 320b, 420b) which is disposed opposite the first end (230) of the second cap (210, 320b, 420b) defines a socket, which receives therein the second rod receiver (140) of the second bone fixation assembly.

3. The reinforcing cross connector apparatus of claim 2, wherein:
the connecting element includes a first link portion (324, 424) extending outwardly away from an outer surface of the first cap (320a, 420a);
a second link portion (324, 424) extending outwardly away from an outer surface of the second cap (320b, 420b); and
a fastener (328, 428)which links the first link portion (324, 424) of the first cap (320a, 420a) to the second link portion (324, 424) of the second cap (320b, 420b).

4. The reinforcing cross connector apparatus of claim 3, wherein the second link portion (324) of the second cap (320b, 420b) extends over the first link portion (324) of the first cap (320a, 420a).

5. The reinforcing cross connector apparatus of claim 4, wherein
the first link portion (324) includes a threaded hole (327) defined therein and the second link portion (324) includes an elongated slot (326) defined therein, and
at least a portion of the elongated slot (326) is aligned over the threaded hole (327) to form a through-passage which receives the fastener (328).

6. The reinforcing cross connector apparatus of claim 4 or 5, wherein the fastener (328) includes a fastener head (328a) and a threaded shaft (329) having a thread (329a) which matingly engages the threaded hole (327) defined in the first link portion (324).

7. The reinforcing cross connector apparatus of anyone of claims 4 to 6, wherein
an annular groove (321) is defined within an inner surface of the first cap (320a, 420a), and
the upper portion (356) of the first locking mechanism includes a flange (351) which is received by the annular groove (321).

8. The reinforcing cross connector apparatus of anyone of claims 3 to 7, wherein at least one of the first and second link portions (324, 324) comprises a pivoting hinge (425).

9. The reinforcing cross connector apparatus of claim 7, wherein
a U-shaped channel (325) is defined within an inner surface of the second cap (320b, 420b), and
the upper portion (356) of the second locking mechanism includes a flange (351) which is received by the U-shaped channel (325).

10. The reinforcing cross connector apparatus of claim 8, wherein at least one of the first link portion (324) and the second link portion (324) comprises a fixed section (424a) and a pivoting section (424b) joined together by a pin connection (425a).

## Patentansprüche

1. Verstärkende Querverbindungsvorrichtung (100, 200, 300), welche eine erste Knochenfixierungsanordnung mit einer zweiten Knochenfixierungsanordnung verbindet, wobei die erste Knochenfixierungsanordnung eine erste Knochenschraube (130) und eine erste Stangenaufnahme (140) aufweist, welche einen Kopf der ersten Knochenschraube (130) umgreift, die zweite Knochenfixierungsanordnung eine zweite Knochenschraube (130) und eine zweite Stangenaufnahme (140) aufweist, welche einen Kopf der zweiten Knochenschraube (130) umgreift, und die verstärkende Querverbindungsvorrichtung (100, 200, 300) aufweist:
einen ersten Aufsatz (210, 320a, 420a), der die äußere Oberfläche der ersten Stangenaufnahme (140) umgreift und verbindet, der erste Aufsatz (210, 320a, 420a) einen ersten Verriegelungsmechanismus aufweist, wobei ein unterer Abschnitt (250, 354) des ersten Verriegelungsmechanismus in eine innere Oberfläche der ersten Stangenaufnahme (140) greift, und ein oberer Abschnitt (290, 356) des ersten Verriegelungsmechanismus in eine innere Oberfläche des ersten Aufsatzes (210, 320a, 420a) greift;
einen zweiten Aufsatz (210, 320b, 420b), der die äußere Oberfläche der zweiten Stangenaufnahme (140) umgreift und verbindet, der zweite Aufsatz (210, 320b, 420b) einen zweiten Verriegelungsmechanismus aufweist, wobei ein unterer Abschnitt (250, 354) des zweiten Verriegelungsmechanismus in eine innere Oberfläche der zweiten Stangenaufnahme (140) greift, und ein oberer Abschnitt (290, 356) des zweiten Verriegelungsmechanismus in eine innere Oberfläche des zweiten Aufsatzes (210, 320b, 420b) greift; und
ein Verbindungselement (280, 324), welches den ersten Aufsatz (210, 320a, 420a) und den zweiten Aufsatz (210, 320b, 420b) miteinander verbindet, wobei der erste Verriegelungsmechanismus den ersten Aufsatz (210, 320a, 420a) und das Verbindungselement (280, 324) gegenüber der ersten Knochenfixierungsanordnung sichert
und der zweite Verriegelungsmechanismus den zweiten Aufsatz (210, 320b, 420b) und das Verbindungselement (280, 324) gegenüber der zweiten Knochenfixierungsanordnung sichert.

2. Verstärkende Querverbindungsanordnung nach Anspruch 1, wobei
der erste Aufsatz (210, 320a, 420a) eine darin definierte Bohrung (222, 323) aufweist, eine Längsachse (L) sich durch die Bohrung (222, 323) des ersten Aufsatzes (210, 320a, 420a) erstreckt, und ein erstes Ende (230) des ersten Aufsatzes (210, 320a, 420a) den ersten Verrieglungsmechanismus darin aufnimmt;
der zweite Aufsatz (210, 320b, 420b) eine darin definierte Bohrung (222, 323) aufweist, eine Längsachse (L) sich durch die Bohrung (222, 323) des zweiten Aufsatzes (210, 320b, 420b) erstreckt, und ein erstes Ende (230) des zweiten Aufsatzes (210, 320b, 420b) den zweiten Verriegelungsmechanismus darin aufnimmt;
das Verbindungselement (280, 324), welches das erste Ende (230) des ersten Aufsatzes (210, 320a, 420a) und das erste Ende (230) des zweiten Aufsatzes (210, 320b, 420b) miteinander verbindet; und
ein zweites Ende (240) des ersten Aufsatzes (210, 320a, 420a), welches gegenüber dem ersten Endes (230) des ersten Aufsatzes (210, 320a, 420a) angeordnet ist, eine Aushöhlung darstellt, welche die erste Stangenaufnahme (140) der ersten Knochenfixierungsanordnung darin aufnimmt, und ein zweites Ende (240) des zweiten Aufsatzes (210, 320b, 420b), welches gegenüber dem ersten Ende (230) des zweiten Aufsatzes (210, 320b, 420b) angeordnet ist, eine Aushöhlung darstellt, welche die zweite Stangenaufnahme (140) der zweiten Knochenfixierungsanordnung darin aufnimmt.

3. Verstärkende Querverbindungsanordnung nach Anspruch 2, wobei das Verbindungselement aufweist:
einen ersten Verbindungsabschnitt (324, 424), welcher sich nach außen, weg von einer äußeren Oberfläche des ersten Aufsatzes (320a, 420a), erstreckt;
einen zweiten Verbindungsabschnitt (324, 424) aufweist, welcher sich nach außen, weg von der äußeren Oberfläche des zweiten Aufsatzes (320b, 420b) erstreckt; und
ein Fixiermittel (328, 428), welches den ersten Verbindungsabschnitt (324, 424) des ersten Aufsatzes (320a, 420a) mit dem zweiten Verbindungsabschnitt (324, 424) des zweiten Aufsatzes (320b, 420b) verbindet.

4. Verstärkende Querverbindungsanordnung nach Anspruch 3, wobei sich der zweite Verbindungsabschnitt (324) des zweiten Aufsatzes (320b, 420b) über den ersten Verbindungsabschnitt (324) des ersten Aufsatzes (320a, 420a) erstreckt.

5. Verstärkende Querverbindungsanordnung nach Anspruch 4, wobei
der erste Verbindungsabschnitt (324) eine darin definierte Gewindebohrung (327) aufweist,
der zweite Verbindungsabschnitt (324) einen darin definierten Längsschlitz (326) aufweist, und
mindestens ein Abschnitt des Längsschlitzes (326) über der Gewindebohrung (327) mit dieser fluchtet, um einen Durchlass zu bilden, welcher das Fixiermittel (328) aufnehmen kann.

6. Verstärkende Querverbindungsanordnung nach Anspruch 4 oder 5, wobei das Fixiermittel (328) einen Fixierkopf (328a) und einen Gewindeschaft (329) mit einem Gewinde (329a) aufweist, welches mit der Gewindebohrung (327) des ersten Verbindungsabschnitts (324) passend in Eingriff ist.

7. Verstärkende Querverbindungsanordnung nach einem der Ansprüche 4 bis 6, wobei
eine ringförmige Nut (321) innerhalb einer inneren Oberfläche des ersten Aufsatzes (320a, 420a) definiert ist, und
der obere Abschnitt (356) des ersten Verriegelungsmechanismus einen Flansch (351) aufweist, welcher in der ringförmigen Nut (321) aufgenommen wird.

8. Verstärkende Querverbindungsanordnung nach einer der Ansprüche 3 bis 7, wobei mindestens einer von dem ersten und zweiten Verbindungsabschnitt (324, 324) ein schwenkbares Gelenk (425) aufweist.

9. Verstärkende Querverbindungsanordnung nach Anspruch 7, wobei
ein U-förmiger Kanal (325) innerhalb einer inneren Oberfläche des zweiten Aufsatzes (320b, 420b) definiert ist, und
der obere Abschnitt (356) des zweiten Verriegelungsmechanismus einen Flansch (351) aufweist, welcher durch den U-förmigen Kanal (325) aufgenommen wird.

10. Verstärkende Querverbindungsanordnung nach Anspruch 8, wobei mindestens einer von dem ersten Verbindungsabschnitt (324) und dem zweiten Verbindungsabschnitt (324) einen starren Abschnitt (424a) und einen schwenkbaren Abschnitt (424b) aufweist, welche durch eine Bolzenverbindung (425a) zusammengefügt sind.

## Revendications

1. Appareil formant connecteur de renforcement en croix (100, 200, 300) qui raccorde un premier ensemble de fixation osseuse à un second ensemble de fixation osseuse, le premier ensemble de fixation osseuse ayant une première vis à os (130) et un premier récepteur de tige (140) englobant une tête de la première vis à os (130), le second ensemble de fixation osseuse ayant une seconde vis à os (130) et un second récepteur de tige (140) englobant une tête de la seconde vis à os (130), l'appareil formant connecteur de renforcement en croix (200, 300, 400) comprenant :
un premier capuchon (210, 320a, 420a) englobant et mettant en prise une surface externe du premier récepteur de tige (140), le premier capuchon (210, 320a, 420a) ayant un premier mécanisme de blocage, dans lequel une partie inférieure (250, 354) du premier mécanisme de blocage met en prise une surface interne du premier récepteur de tige (140) et une partie supérieure (290, 356) du premier mécanisme de blocage met en prise une surface interne du premier capuchon (210, 320a, 420a) ;
un second capuchon (210, 320b, 420b) englobant et mettant en prise une surface externe du second récepteur de tige (140), le second capuchon (210, 320b, 420b) ayant un second mécanisme de blocage, dans lequel une partie inférieure (250, 354) du second mécanisme de blocage met en prise une surface interne du second récepteur de tige (140) et une partie supérieure (290, 356) du second mécanisme de blocage met en prise une surface interne du second capuchon (210, 320b, 420b) ; et
un élément de raccordement (280, 324) qui relie le premier capuchon (210, 320a, 420a) et le second capuchon (210, 320b, 420b) ensemble, le premier mécanisme de blocage fixant le premier capuchon (210, 320a, 420a) et l'élément de raccordement (280, 324) au premier ensemble de fixation osseuse, et le second mécanisme de blocage fixant le second capuchon (210, 320b, 420b) et l'élément de raccordement (280, 324) au second ensemble de fixation osseuse.

2. Appareil formant connecteur de renforcement en croix selon la revendication 1, dans lequel :
le premier capuchon (210, 320a, 420a) comprend un alésage (222, 323) défini à l'intérieur de ce dernier, un axe longitudinal (L) s'étend à travers l'alésage (222, 323) du premier capuchon (210, 320a, 420a), et une première extrémité (230) du premier capuchon (210, 320a, 420a) reçoit le premier mécanisme de blocage à l'intérieur de cette dernière ;
le second capuchon (210, 320b, 420b) comprend un alésage (222, 323) défini à l'intérieur de ce dernier, un axe longitudinal (L) s'étend à travers l'alésage (222, 323) du second capuchon (210, 320b, 420b), et une première extrémité (230) du second capuchon (210, 320b, 420b) reçoit le second mécanisme de blocage à l'intérieur de cette dernière ;
l'élément de raccordement (280, 324) reliant la première extrémité (230) du premier capuchon (210, 320a, 420a) et la première extrémité (230) du second capuchon (210, 320b, 420b) ensemble ; et
une seconde extrémité (240) du premier capuchon (210, 320a, 420a) qui est disposée à l'opposé de la première extrémité (230) du premier capuchon (210, 320a, 420a) définit une douille, qui reçoit à l'intérieur de cette dernière le premier récepteur de tige (140) du premier ensemble de fixation osseuse, et une seconde extrémité (240) du second capuchon (210, 320b, 420b) qui est disposée à l'opposé de la première extrémité (230) du second capuchon (210, 320b, 420b) définit une douille, qui reçoit à l'intérieur de cette dernière le second récepteur de tige (140) du second ensemble de fixation osseuse.

3. Appareil formant connecteur de renforcement en croix selon la revendication 2, dans lequel :
l'élément de raccordement comprend une première partie de liaison (324, 424) s'étendant vers l'extérieur à distance d'une surface externe du premier capuchon (320a, 420a) ;
une seconde partie de liaison (324, 424) s'étendant vers l'extérieur à distance d'une surface externe du second capuchon (320b, 420b) ; et
une fixation (328, 428) qui relie la première partie de liaison (324, 424) du premier capuchon (320a, 420a) à la seconde partie de liaison (324, 424) du second capuchon (230b, 420b).

4. Appareil formant connecteur de renforcement en croix selon la revendication 3, dans lequel la seconde partie de liaison (324) du second capuchon (320b, 420b) s'étend sur la première partie de liaison (324) du premier capuchon (320a, 420a).

5. Appareil formant connecteur de renforcement en croix selon la revendication 4, dans lequel :
la première partie de liaison (324) comprend un trou fileté (327) défini à l'intérieur de cette dernière et la seconde partie de liaison (324) comprend une fente allongée (326) définie à l'intérieur de cette dernière, et
au moins une partie de la fente allongée (326) est alignée sur le trou fileté (327) afin de former un passage débouchant qui reçoit la fixation (328).

6. Appareil formant connecteur de renforcement en croix selon la revendication 4 ou 5, dans lequel la fixation (328) comprend une tête de fixation (328a) et un arbre fileté (329) ayant un filetage (329a) qui met en prise par couplage le trou fileté (327) défini dans la première partie de liaison (324).

7. Appareil formant connecteur de renforcement en croix selon l'une quelconque des revendications 4 à 6, dans lequel :
une rainure annulaire (321) est définie à l'intérieur d'une surface interne du premier capuchon (320a, 420a), et
une partie supérieure (356) du premier mécanisme de blocage comprend une bride (351) qui est reçue par la rainure annulaire (321).

8. Appareil formant connecteur de renforcement en croix selon l'une quelconque des revendications 3 à 7, dans lequel au moins l'une des première et seconde parties de liaison (324, 324) comprend une charnière pivotante (425).

9. Appareil formant connecteur de renforcement en croix selon la revendication 7, dans lequel :
un canal en forme de U (325) est défini à l'intérieur d'une surface interne du second capuchon (320b, 420b), et
la partie supérieure (356) du second mécanisme de blocage comprend une bride (351) qui est reçue par le canal en forme de U (325).

10. Appareil formant connecteur de renforcement en croix selon la revendication 8, dans lequel au moins l'une parmi la première partie de liaison (324) et la seconde partie de liaison (324) comprend une section fixe (424a) et une section pivotante (424b) assemblées ensemble par un raccordement à broche (425a).
